# EUROPEAN PATENT SPECIFICATION

(11) **EP 2 363 083 B1**
(45) Date of publication and mention of the grant of the patent: **12.06.2013**
(21) Application number: 10155104.2
(22) Date of filing: 01.03.2010
(51) Int. Cl.: A61B 17/17, A61B 19/00, A61F 2/46

(54) **Computer assisted surgery system**
Computergestütztes chirurgisches System
Système de chirurgie assistée par ordinateur

(43) Date of publication of application: 07.09.2011
(73) Proprietor: Stryker Trauma GmbH, 24232 Schönkirchen/Kiel (DE)
(72) Inventor: Blau, Arno, 9620, Lichtensteig (CH); Kohnen, Michael, Dr., 79423, Heitersheim (DE); Simon, Bernd, 24107, Kiel (DE); Müller-Daniels, Holger, 24214, Gettorf (DE)
(74) Representative: Schmitz, Alexander

(56) References cited:
- EP-A1- 1 523 950
- WO-A1-2009/087214
- WO-A2-2005/087125
- US-A1- 2004 243 148
- US-A1- 2005 065 617

## Description

### FIELD OF THE INVENTION

The present invention relates to a computer assisted surgery system for operating a computer assisted surgery system, and in particular to a computer assisted surgery system for operating a computer assisted surgery system providing a virtual representation of a medical device to provide an easier application of the medical device, such as an implant or the like.

### BACKGROUND OF THE INVENTION

Fractures of the femoral neck, for example, may be treated by intramedullary nailing. In such treatments, a nail for intramedullary nailing typically comprises at least one bore hole for receiving a bone screw. The nail is generally introduced in the longitudinal direction of the femur, wherein the bone screw laterally extends at a certain angle with respect to the neck of the femur when the bone screw is received within the at least one bore hole. A certain problem of the surgeon is to predict the future or implanted position of such a nail or implant or parts therof. In the past, the operator has acted in a trial and error manner to obtain a more or less optimum position of the implant. However, this may lead to a longer duration of the operation which may lead to higher stress for the patient. Further, for each trial, at least one X-ray image (e.g. a fluoroshot) is generally necessary in order to check the present position of the implant in order to evaluate its position.

In recent times, computer assisted surgery for dynamic hip screw had been described by Amir Herman et al. in The International Journal of Medical Robotics and Computer Assisted Surgery, Dec. 29, 2008; Volume 5, pages 45-50, describes a computer assisted surgery system using an image analysis technology in order to measure three-dimensional distances, visualize implant templates, and view a guided trajectory on standard fluoroscopy. A guiding system combines a set of X-ray opaque markers incorporated into transparent hardware as an aiming, positioning, and referring device. This device is attached to a guide wire. Fluoroscopic images are obtained by the surgeon and then are processed by an image processing engine which calculates a three-dimensional orientation relative to a C-arm and a drill trajectory in the image.

Further, a process for the acquisition of information intended for the insertion of a locking screw into an orifice of an endomedullary device is described in EP 1 491 151 B1. This document describes a process for the acquisition of information intended for the insertion of a locking screw into a distal locking hole of an endomedullary device. The described process includes taking two images of different orientations of the distal part of the endomedullary device using a radioscopic unit, acquisition of projection parameters, especially the position of the X-ray source and the projection plane of each image by locating a reference frame fixed on the endomedullary device and optionally another reference frame fixed on the radioscopic unit. The process further includes correcting any distortion of the images, segmenting the distal part of the endomedullary device in each image and calculating the attributes relating to the position of the device and to that of the holes, wherein the attributes comprise at least the contours of the device, its centre of gravity and its principal axis. Further, the process includes constructing the projection cone of the distal part of the device for each image, determining the intersection of the two projection cones, modelling of the endomedullary device on modelling and of the centres of gravity of the holes determined on the images, determining the orientation of the locking orifice in an iterative manner, and guiding of a drill tool.

WO 2009/087214 describes a method for assisting surgery and a correspondingly adapted system, the method including the steps of imaging a region of interest, processing the image data to achieve a three dimensional visualization of the region of interest, associating a virtual representation of a medical devise with the region of interest and displaying the resulting image.

### SUMMARY OF THE INVENTION

The present invention provides a computer assisted surgery system according to claim 1 allowing a simple and fast positioning of a medical device to be applied, such as an implant, for example.

There is provided a method for operating a computer assisted surgery system, the method comprising positioning of a reference body in relation to an anatomical structure, the reference body virtually representing a position of a medical device to be applied to the anatomical structure, detecting a position of the reference body in relation to the anatomical structure, superimposing the anatomical structure with a virtual representation of a medical device to be applied based on the detected position of the reference body in relation to the anatomical structure, providing rules for allowable ranges for applying the medical device in relation to the anatomical structure, modifying the position of the reference body, and optimizing the virtual position of the medical device to be applied with respect to the anatomical structure so as to obtain a best fit with respect to the rules for allowable ranges.

Thus, by using a virtual representation of a medical device to be applied, a future position of a real medical device can be predicted without the need of inserting this medical device during the phase of determining the final desired position of the medical device. Thus, the position of the medical device can be virtually optimized before inserting the medical device. This may lead to reducing the stress for the patient with respect to an incision and X-ray impact. Optimizing may include finding of the optimal location, orientation and geometry of the medical device, i.e. the implant. This optimizing may take place supported by a computer device. The reference body may be a particular add-on element as well as a medical tool having a unique geometry to identity the position thereof in imaging.

The position of a medical device includes dimensions, location, and orientation of the medical device.

Thus, the medical device can be virtually represented considering all relevant information with respect to an anatomical structure of the patient. Position may also be the geometry of the medical device, in particular out of a predetermined variety of medical devices.

Detecting the positioned reference body in relation to an anatomical structure comprises taking two two-dimensional images from different angles and generating a three-dimensional representation based on the two two-dimensional images, and determining a spatial position of the reference body in relation to the anatomical structure based on the three-dimensional representation. The two 2-dimensional images may include the anatomical structure receiving the medical device. The position of the medical device in relation to the anatomical structure may be based on the 3-dimensional representation.

Thus, the anatomical structure as well as the virtual implant or the virtual medical device to be applied can be represented in a three-dimensional manner in order to give an overview over the correct positioning and dimensioning of the medical device to be applied.

Modifying may comprise rotating and/or displacing the reference body.

Thus, the reference body representing the position of the medical device is virtually positioned with respect to the anatomical structure so as to find out the optimized position of the future positioned medical device within the rules provided, wherein the rules provide the allowable ranges for applying the medical device in relation to the anatomical structure.

Modifying may comprise selecting the medical device out of a predetermined group of a variety of medical devices.

Thus, for meeting the rules for allowable ranges, also the dimensions of the medical devices can be selected out of a predetermined group in order to find out an optimal implant type to be implanted, for example. The various types may have various geometries, e.g. lengths, inclination angles, and other geometric properties, corresponding to the various anatomical properties.

The method of operating a computer assisted surgery system further comprises imaging the superposition of the anatomical structure and the virtual representation of the medical device to be inserted.

Thus, the surgeon is capable of in-situ controlling and monitoring of the ongoing process of the computer assisted surgery system, which may be of relevance when finally deciding whether the optimization is sufficient and to provide a final check by the surgeon in person.

The position of the medical device to be applied is remote from the reference body. Preferably, the medical device is an implant. Preferably, the reference body is mountable to a medical tool, such as an aiming tool, for example.

Thus, the reference body does not have to be provided in the immediate vicinity of the medical device. Implants may be virtually represented that are not in direct vicinity of the reference body. This is particularly relevant for implants that have a final remote position with respect to an opening location of the incision. Further, also sub-implants can be virtually represented, such as a bone screw of an intramedullary nail, for example, when providing the reference body to an aiming tool.

There is provided a program element, which, when being executed by a processor is adapted to carry out the inventive method for operating a computer assisted surgery system.

There is provided a computer readable medium having stored the inventive program element.

Thus, the method for operating a computer assisted surgery system can be carried out on a computer and a computer program, respectively.

According to still yet another embodiment of the invention, there is provided a computer assisted surgery system comprising a reference body in relation to an anatomical structure, the reference body virtually representing a position of a medical device to be applied to the anatomical structure, a detector device being adapted for detecting a position of the reference body in relation to the anatomical structure, a database including virtual medical device information and a computation device being adapted for superimposing the anatomical structure with a virtual representation of a medical device to be applied, based on an output of the detector device and modifying the position of the reference body and optimizing the virtual position of the medical device to be applied with respect to the anatomical structure so as to obtain the best fit with respect to predetermined rules for allowable ranges for applying the medical device in relation to the anatomical structure.

Such a computer assisted surgery system allows to predict the future position of a medical device to be applied, such as an implant, for example, without the need for a trial and error procedure of a surgeon in order to meet the predetermined rules for allowable ranges for applying the medical device in relation to the anatomical structure, which rules may be provided in form of required distances, for example, to the surface of the bone, particular inclination angles between for example the longitudinal direction of the femur and the orientation of a femoral neck, and the like.

According to an embodiment of the invention, the data base includes a plurality of data sets for the medical device, wherein the data sets represent a variety of medical devices.

Thus, not only the orientation and the location of the medical device can be determined in order to meet the predetermined rules for allowable ranges, but also the dimensions of the medical device as such. This may be of relevance in particular when having a wide variety of anatomies requiring different dimensions with respect to the length, the diameter and particular angles of for example an intramedullary nail and the respective bone screws. According to an embodiment of the invention, the medical device is an implant.

According to still yet another embodiment of the invention, the system further comprises a medical tool being adapted for positioning the implant, wherein the reference body is mountable in a predefined manner to the medical tool.

Thus, the reference body may be used for representing an intramedullary nail, which is still not implanted in order to find the correct position of the intramedullary nail as well as the correct position of the future implanted bone screw before having inserted the intramedullary nail. However, the intramedullary nail may be considered as a reference body, as the intramedullary nail may have a unique form representing also the future position of a bone screw to be inserted. Thus, when having inserted the intramedullary nail being mounted to the medical tool, from the geometry of the intramedullary nail, the future position, i.e. the location, the dimension, and the orientation of the bone screw can be determined. It should be noted that the intramedullary nail can be used as a reference body representing the future position of a bone screw, for example. However, also a separate reference body can be used for representing for example the intramedullary nail, wherein such reference body may be fixed in a predetermined position to the medical tool or the nail in order to represent and predict the future position of the intramedullary nail. When knowing the geometry and orientation of the intramedullary nail, also the future position of a bone screw can be predicted by evaluating the reference body mounted to the medical tool.

It should be noted that the above described embodiments apply also for the method of operating the computer assisted surgery system, the computer assisted surgery system, the program element as well as the computer readable medium. bone screw can be predicted by evaluating the reference body mounted to the medical tool.

It should be noted that the above described embodiments of the invention apply also for the method of operating the computer assisted surgery system, the computer assisted surgery system, the program element as well as the computer readable medium.

It should also be noted that the above feature may also be combined. The combination of the above features may also lead to synergetic effects, even if not explicitly described in detail.

These and other aspects of the present invention will become apparent from and elucidated with reference to the embodiments described hereinafter.

### BRIEF DESCRIPTION OF THE DRAWINGS

The present invention will be better understood on reading the following detailed description of non-limiting embodiments thereof, and on examining the accompanying drawings, in which:

Fig. 1 illustrates a computer assisted surgical system.

Fig. 2 illustrates a medical tool having fixed thereon a reference body and an implant including an intramedullary nail and a bone screw.

Fig. 3 illustrates the implant being separated from the medical tool shown in Fig. 2.

Fig. 3a illustrates the reference body shown in Fig. 3.

Fig. 3b illustrates an end view of the reference body shown in Fig. 3.

Fig. 4a illustrates a virtual representation of the implant.

Fig. 4b illustrates a real position of the implant.

Fig. 5 illustrates a virtual representation of a bone screw and a real position of a nail.

Fig. 6 illustrates a virtual representation of a bone screw and a nail.

Fig. 7a illustrates a deviation to a first direction of a virtual nail and a virtual screw as shown by the arrows provided.

Fig. 7b illustrates a deviation to a second direction of a virtual nail and a virtual screw as shown by the arrows provided.

Fig. 7c illustrates a correct position of a virtual nail and a virtual screw.

Fig. 7d illustrates a final position of a real nail and a real screw.

Fig. 8a illustrates a deviation to a first direction of a real nail and a virtual screw as shown by the arrows provided.

Fig. 8b illustrates a deviation to a second direction of a real nail and a virtual screw as shown by the arrows provided.

Fig. 8c illustrates a correct position of a real nail and a virtual screw.

Fig. 8d illustrates a final position of a real nail and a real screw.

Fig. 9a illustrates a virtual representation of a variety of different geometries, i.e. inclination angles of a screw with respect to a nail.

Fig. 9b illustrates a virtual representation of a variety of different geometries, i.e. inclination angles of a screw with respect to a nail.

Fig. 10 illustrates a virtual representation of a variety of different geometries, i.e. lengths of a screw with respect to a real nail.

Fig. 11 illustrates a schematic flow diagram of the method for operating a computer assisted surgical system.

### DETAILED DESCRIPTION

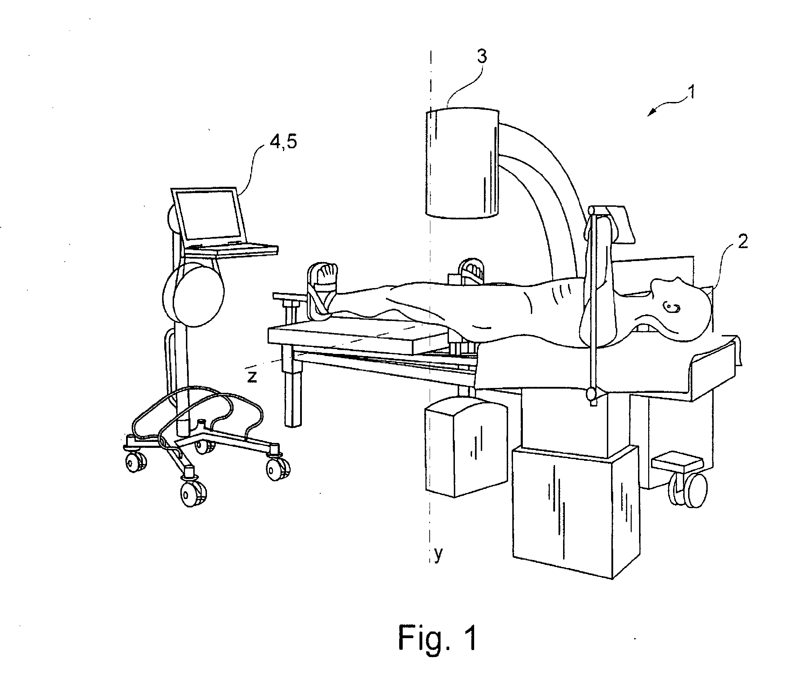

Fig. 1 illustrates a computer assisted surgery system 1. A patient 2 can be positioned in or on the computer assisted surgery system so that application of a medical device to be applied, such as an implant, for example, can be assisted. The computer assisted surgery system of Fig. 1 illustrates a configuration for the implantation of the intramedullary nail in the femur of the patient 2. For this purpose, an imaging device 3 is provided in order to deliver images from the location of the anatomical structure for which the application of the implant is intended. The computer assisted surgery system further comprises a display unit 4 as well as a computation unit 5 so that the correct position of the medical device and implant, respectively, can be computed and displayed on the display unit. Thus, the surgeon receives assistance in applying an intramedullary nail and a respective bone screw, for example, so that the total incision time can be reduced and the position of the implant can be improved.

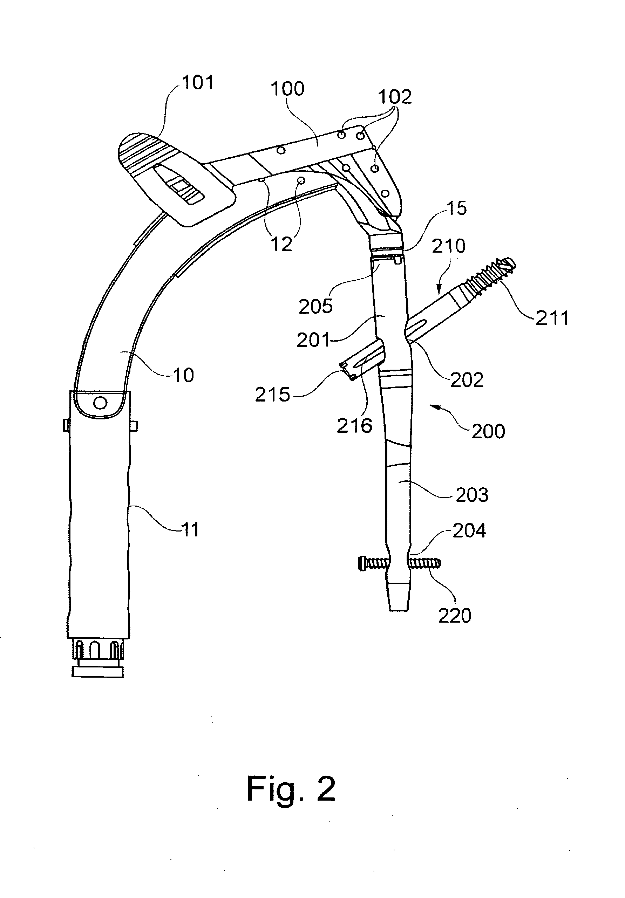

Fig. 2 illustrates a medical application tool 10 in form of an aiming tool. The aiming tool comprises a finger grip 11 and a coupling portion 15 for coupling a medical device 200 to be applied. This medical device in Fig. 2 is an intramedullary nail 200. This intramedullary nail has an upper portion 201 also comprising a coupling portion 205 for coupling the intramedullary nail to the coupling portion 15 of the medical tool 10. In the embodiment shown in Fig. 2, the intramedullary nail 200 comprises an orifice 202 provided in the upper shaft portion 201 of the intramedullary nail. This orifice 202 serves for receiving a bone screw 210. The intramedullary nail further comprises a lower shaft portion 203 comprising a further orifice 204 for receiving a distal fixation screw 220. The bone screw 210 is designed to extend into the neck of femur bone. For this purpose, the bone screw 210 is provided with a gear shaft 211 for a fixation in the bone material. Further, the bone screw is provided with a fixation arrangement 216, so that the bone screw can be fixed within the intramedullary nail 200. This fixation can be carried out by an internal screw along the longitudinal extension of the intramedullary nail in the upper shaft portion 201 so as to fix the position of the bone screw 210 with respect to the intramedullary nail 200. Further, the bone screw 210 can be provided with a receptacle 215 for receiving a respective tool, e.g. a screw driver, for turning the bone screw 210 into the bone, for example the femoral neck.

In Fig. 2, a reference body 100 is fixed in a predetermined manner onto the medical tool 10. The reference body comprises a finger grip 101 for an easier fixation and positioning of the reference body. The reference body further comprises a plurality of fiducial markers 102. These markers are distributed over the reference body in a predefined manner in order to give a unique representation in any two-dimensional projection, so that a single fluoroshot image may be sufficient for determining the unique 3D-position of the reference body 100. As the reference body 100 is fixed to the medical tool 10 in a predefined manner, also with respect to the intramedullary nail 200, the known orientation, location, and in general position of the reference body 100 at the same time represents the position of the intramedullary nail 200. In case, the geometry of the intramedullary nail 200 is known, also the position of the bone screw 210 and the distal locking screw 220 is predefined at least for the direction of the longitudinal extension thereof. Thus, the positioning of the medical tool 10 together with the reference body 100 allows for determining the position of the intramedullary nail 200 as well as at least the longitudinal extension direction of the bone screw 210 and the distal locking screw 220, irrespective of the visibility of the intramedullary nail in a fluoroshot image, for example. In order to determine whether the reference body 100 is correctly positioned with respect to the medical tool 10, also the medical tool 10 can be provided with a plurality of fiducial markers 12, so that the correct position of the reference body with respect to the medical tool 10 can be determined by evaluating a single fluoroshot image.

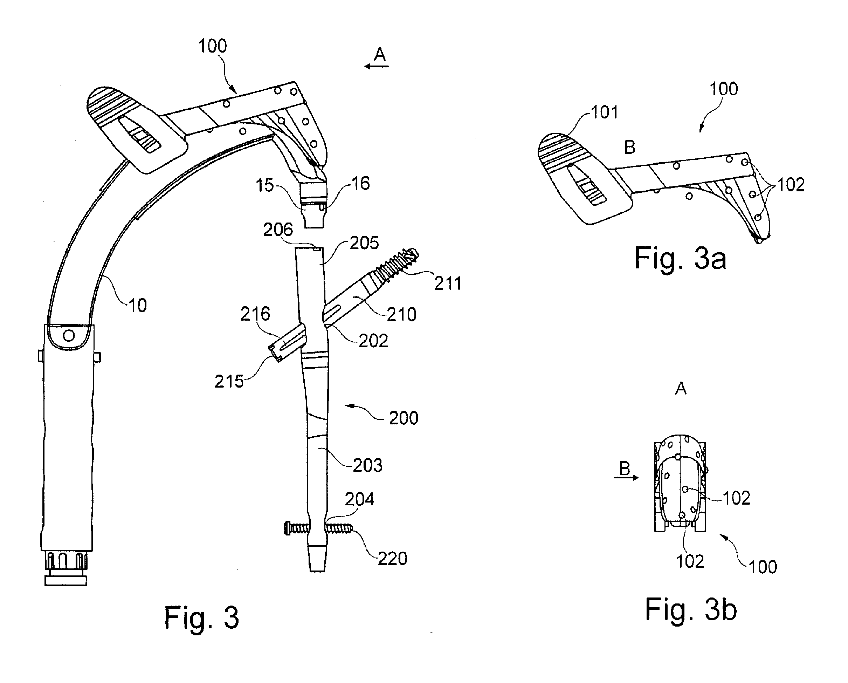

Fig. 3 illustrates the single elements of the implant portion and the tool portion shown in Fig. 2. Fig. 3 illustrates the intramedullary nail 200 in a released manner with respect to the medical tool 10. The medical tool 10 comprises a coupling portion 15 having a unique matching pattern 16 in form of for example noses for receiving a respective counterpart of the intramedullary nail 206, 205. Thus, a unique matching position of the intramedullary nail 200 with respect to the medical tool 10 can be provided, so that it can be ensured that the reference body 100 can be used for pre-definitely representing the intramedullary nail 200.

Fig. 3a illustrates a front view of the reference body 100. The fiducial markers 202 are irregularly distributed over the reference body 100, however, in a predefined manner, so that a single fluoroshot allows a unique determination of the spatial position of the reference body. Fig. 3b illustrates a side view of the reference body being separated from the medical tool 10.

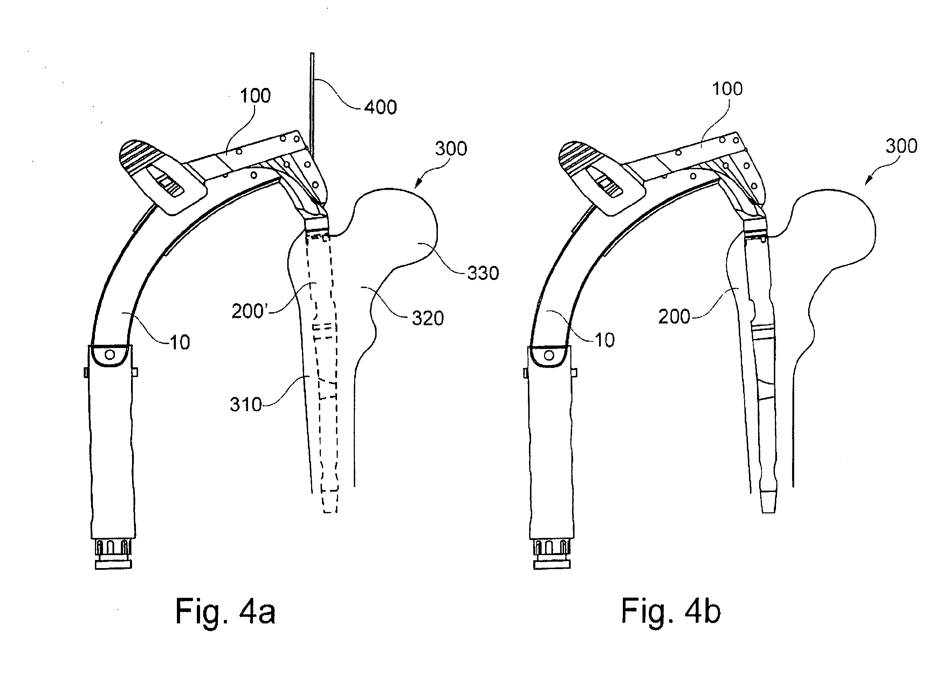

When knowing the position of an intramedullary nail 200 with respect to the reference body, the variation of the position of the reference body, here mounted onto the medical tool 10, can be used to determine a future position of the intramedullary nail, even if the nail is not mounted to the medical tool 10. This can be seen from Fig. 4a. which illustrates an anatomical structure 300 in form of a femur bone having a femur head 330, a femur neck 320 and femur shaft 310. When positioning the medical tool 10 having mounted thereon the reference body 100, a future position of an intramedullary nail can be determined by visualizing a virtual representation of the intramedullary nail 200'. It should be noted that for the following description, the reference numbers with an apostrophe represent a virtual portion of a medical device to be applied, e.g. an implant, wherein the references without an apostrophe represent the real medical device, also when already applied.

When positioning the medical tool 10 onto the top of the femur bone, the modification of the position of the medical tool together with the reference body 100 allows determining a virtual representation of the later applied medical device. When having found the correct position of the medical device to be implanted, as shown in Fig. 4a, a respective guide wire 400 can be applied to the femur bone so as to fix the point of entry, which belongs to an optimal position of the medical device to be applied. Then, the medical tool 10 can be removed while remaining the guide wire 400 at the femur bone 300. Thus, the entry point is fixed in order to apply a drilling device or an awl for opening the respective entering point of the femur bone. After the drilling, the real intramedullary nail 200 can be coupled to the medical tool 10 in order to insert the intramedullary nail into the femur bone 300, in particular the femur shaft 310, as can be seen from Fig. 4b.

The reference body may also be mounted to an awl or bore tool, or to a targeting tool for representing an awl. When providing an awl with a reference body, the future position of the nail can be predicted based on the trajectory of the awl. Thus, the future position of the nail can be determined when producing the bore hole, e.g. by an awl or a drilling tool. In other words, it is possible to determine the future nail position in situ when drilling the hole for the nail.

It should be noted that according to the known geometry of the intramedullary nail and the predefined coupling of the intramedullary nail 200 to the medical tool 10 via the predefined coupling arrangement 205, 206, 15, 16, also the direction of the bone screw 210 is defined as well as the direction of the distal locking screw 220.

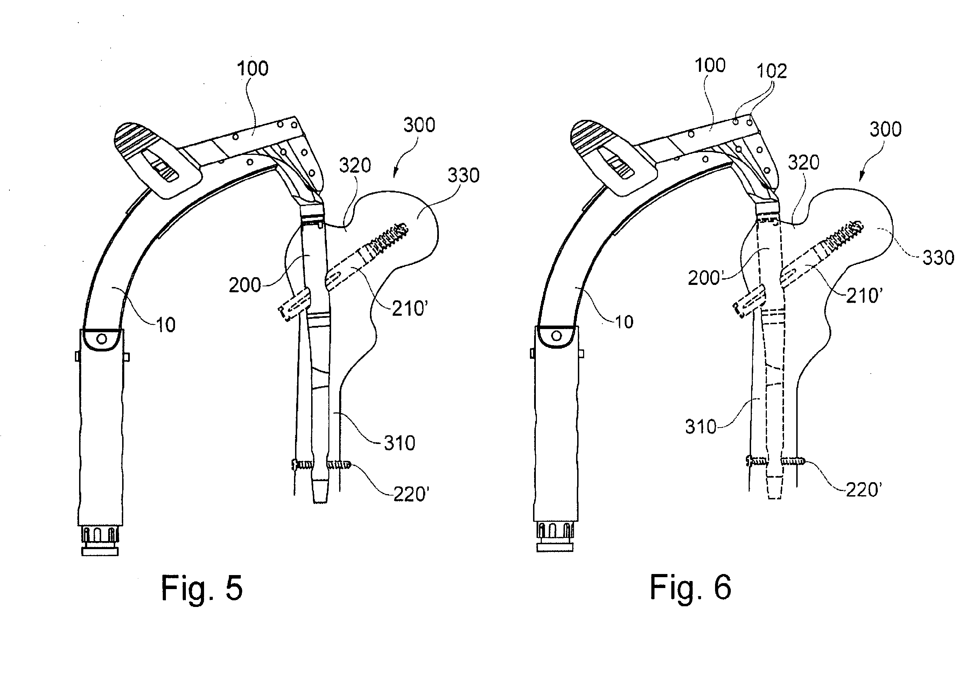

Fig. 5 illustrates starting from Fig. 4b having inserted the real intramedullary nail 200, the virtual representation of the bone screw 210' and the locking screw 220'. Although the exact position of the bone screw as well as the locking screw can be varied, the longitudinal direction and orientation thereof is predefined by the orifices 202, 204 of the intramedullary nail. Thus, when having inserted the intramedullary nail, a virtual representation of the bone screw 210' can be used in order to determine the correct position of the intramedullary nail with respect to a longitudinal translation as well as a rotation with respect to the longitudinal axis of the intramedullary nail. It should be understood, that also the intramedullary nail 200 can be provided with a unique geometry allowing the defined determination of the position of the intramedullary nail, i.e. the location, the dimension, and the orientation thereof. In other words, when using the real intramedullary nail 200 as a reference body, an additional reference body 100 on top of the medical tool may be left out, as the intramedullary nail then may serve as a reference body for the bone screw 210' to be applied as well as a distal locking screw 220' to be applied.

Fig. 6 illustrates a virtual representation of the intramedullary nail 200' together with a virtual representation of the bone screw 210' and the distal locking screw 220'. As the position of the bone screw 210' is defined by the orientation thereof with respect to the intramedullary nail 200', the positioning of the medical tool can be used to find the correct position of the intramedullary nail as well as the bone screw. By repositioning of the medical tool 10, the virtual representation of the intramedullary nail 200' together with the bone screw 210' varies, so that the correct position not only of the intramedullary nail but also of the bone screw can be determined. This allows for example to determine the correct axial displacement of the virtual intramedullary nail 200' in order to find the correct position of a virtual bone screw 210' to maintain certain distances between the bone screw and the bone surface of the femoral neck 320. This will be described in greater detail with respect to the following figures.

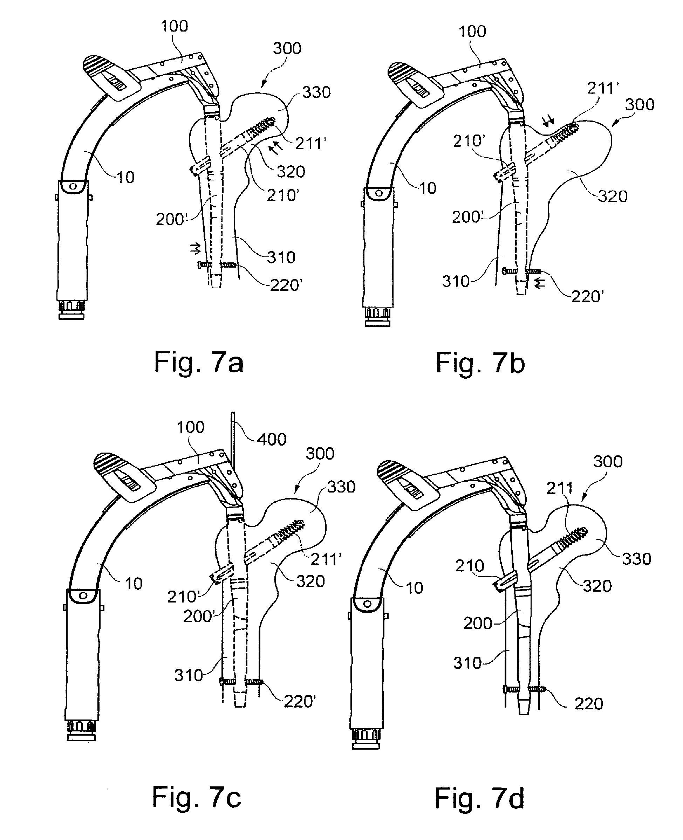

Fig. 7a illustrates a virtual position of the intramedullary nail 200' together with the virtual representation of the bone screw 210'. However, the virtual representation of the implants, the intramedullary nail as well as the bone screw, illustrates a position, which is not sufficient for a final insertion of the intramedullary nail as well as the bone screw, as the distances of the intramedullary nail to the surface of the femur shaft as well as the distance of the bone screw to the surface of the femur neck are too narrow (see arrows). Thus, the position of the medical tool 10 has to be modified in order to find a better positioning.

Fig. 7b illustrates a repositioning, however, this positioning is also not suitable for a final insertion of the implant, as the virtual representation of the intramedullary nail 200' as well as the virtual representation of the bone screw 210' is again too narrow to the surface of the femur shaft 310 and the femur neck 320, respectively. After a further repositioning, according to Fig. 7c, a correct position of the virtual intramedullary nail 200' and a virtual representation of the bone screw 210' is achieved, so that the correct position can be fixed, for example by applying a guide wire 400. After having fixed the correct point of entry, the entire real implant, i.e. the intramedullary nail 200 and the bone screw 210 can be applied to the femur bone 300 in the previously determined position. It should be noted that according to the unique representation of the reference body 100, uniquely representing the intramedullary nail as well as the direction of the bone screw, no further fluoroscopic shots are required between Figs. 7a and 7c. Another fluoroscopic shot may be taken if controlling the final implant position is desired to confirm a successful implantation thereof, as illustrated in Fig. 7d.

As shown in Fig. 7c, in the case where the correct point of entry is determined, the intramedullary nail 200 can be coupled to the medical tool 10 and then can be inserted into the femur shaft 310. Either the intramedullary nail 200 or the reference body 100 or both, the reference body 100 and the intramedullary nail 200, can be used as a reference body 100 in order to virtually represent the bone screw 210' and a virtual representation of the distal locking screw 220'. By repositioning the medical tool 10 together with the intramedullary nail 200, the correct future position of the bone screw 210' can be determined.

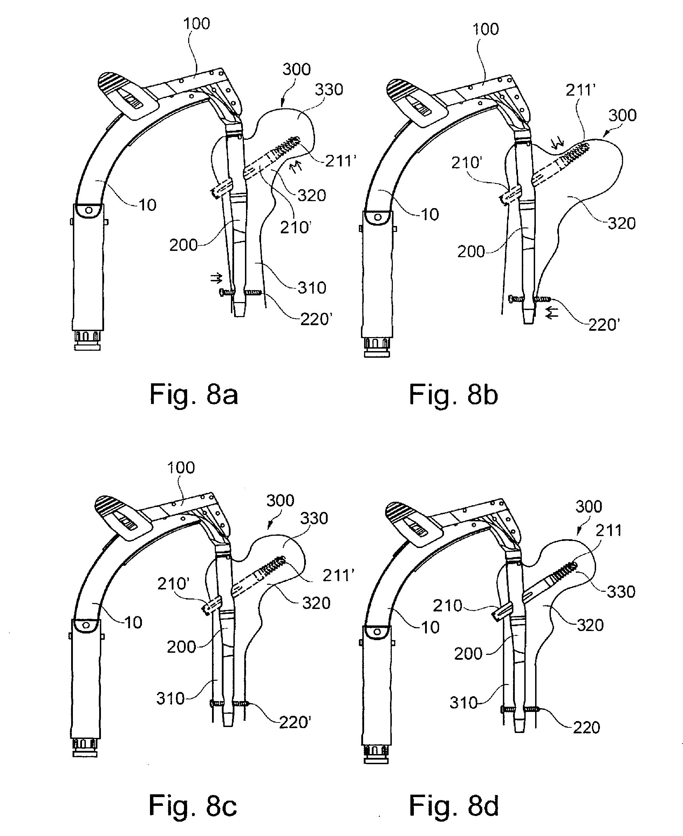

Figs. 8a to 8d illustrate the placement of a virtual representation of a bone screw 210' when having positioned a real nail 200. Fig. 8a illustrates an insufficient position with respect to the low distance between the virtual bone screw 210' to the surface of the femur neck 320 (arrows), whereas Fig. 8b illustrates a counter-positioned insufficient positioning with respect to the other side of the femur neck (arrows). Fig. 8c illustrates a better position of the intramedullary nail 200 with respect to the virtually representation of the bone screw 210', so that the real bone screw 210 can be inserted as shown in Fig. 8d. It should be noted that the reference body 100 can also be used to represent for example a boring tool in order to provide a bore hole into the shaft of the femur neck 310, so that during the drilling process, the correct position of the driller can be monitored without the need for single fluoroshot images during the drilling procedure. The reference body may also represent the nail 200' when drilling a bore hole so that the correct positioning of the nail can be monitored when drilling the hole for receiving the later nail. This monitoring of the drilling procedure is similar to the illustration of Fig. 8a to 8c, wherein the intramedullary nail 200 then is replaced by a driller, whereas the virtual representation of the bone screw 210' may be maintained in order to ensure the correct position of the bone screw 210.

It should be noted that the computer assisted surgery system may also assist in finding a better position or orientation of the reference body 100, 200. This can happen by giving detailed instructions to the surgeon in which the aiming tool direction should be moved to find the correct position. It is also possible to give a haptic feedback to the handgrip of the aiming tool, for example, so that the surgeon can directly recognize in which direction he should move the aiming tool. For this purpose respective actors can be placed to the handle or grip.

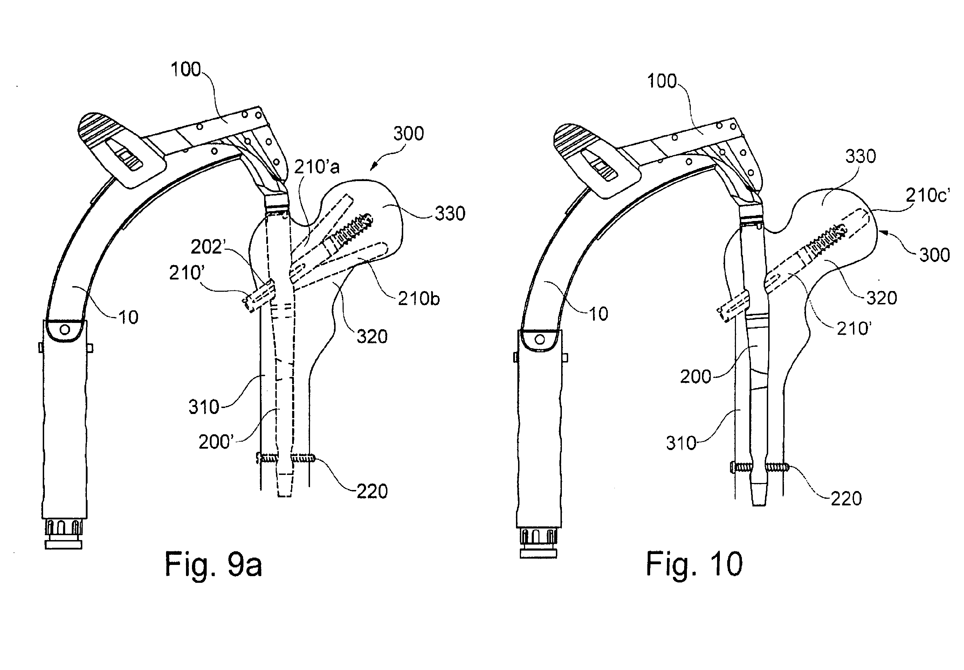

Fig. 9a illustrates the visualization of the virtual representation of an intramedullary nail having a varying geometry of the orifice 202. By selecting a respective virtual intramedullary nail 200', the inclination of the bone screw 210' can be varied to a steeper position 210a' or a less inclined position 210b'. Thus, by virtually representing a variation of possible intramedullary nails allowing different inclination angles of the bone screw 210', the correct type of intramedullary nail can be selected in order to achieve the correct positioning of the later implanted bone screw 210. This selection can be carried out by the computer assisted surgical system when searching for an optimum geometry in the database and proposing the respective type of implant.

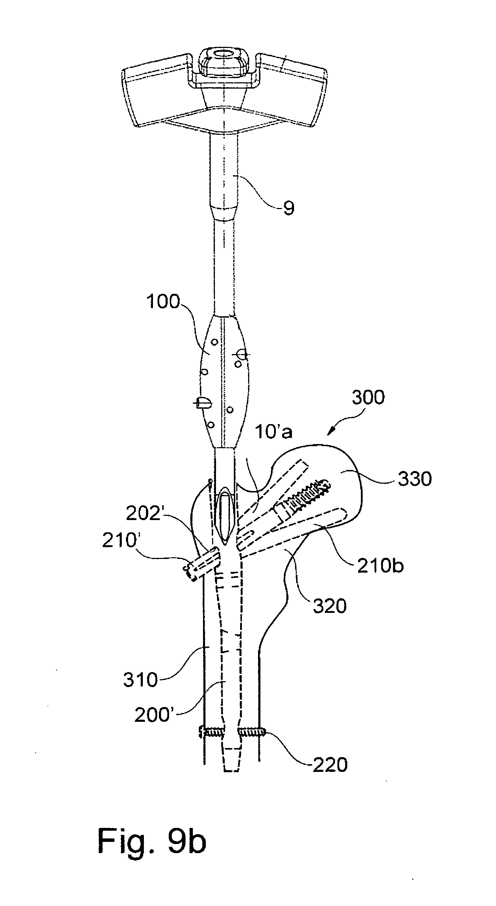

In practice stereotaxis with intra-operative X-ray imaging is used, wherein an awl 9 provided with a reference body 100 may be used to drill a bore hole for the nail, as illustrated in Fig. 9b. The system may detect the reference body 100 of the awl 9 and thus knows the axis of implant 200', e.g. the axis of the gamma nail/intramedullary nail in 3D space. When having entered the bone for a certain distance, the trajectory of the awl 9 is substantially determined. However, slight corrections may be carried out depending on the depth of the awl. By producing two two-dimensional images, e.g. one in the AP-direction and one in the ML-direction, the anatomic structure can be visualized together with the already entered awl. In ML view, the system segments femoral head and thus knows the centre of the femoral head, and is thus able to calculate correct rotation of implant in ML view. In AP view, the system overlays implant with correct axis rotation as calculated in ML, performs automatic segmentation of femoral head, thus calculates the centre of the femoral head (or the Apex). The virtual representation of the nail allows to rotate and/or to translate the reference body so as to find the optimized position for the nail. The system then may virtually move the implant e.g. the nail along the trajectory corresponding to the nail axis until trajectory of bone screw, e.g. for 125° inclination angle between nail 200' and bone screw 210' as default, runs through head centre (or Apex) and displays, in addition to the default type, all other available types of implant. This may be carried out by a software tool. The optimal nail position and optimal nail type may be determined automatically by the computation device based on the available data sets of the data-base so that the surgeon may receive a proposal for the nail position and the nail type, as well as the corresponding bone screw and/or the distal locking screw. Optionally the user may interact with the system to adapt entry depth of nail. Optionally the system may present a 3D reconstruction of whole scenery. The already introduced awl provides for a stable position in the bone so that the future position of the intramedullary nail can be provided. Further, the rigid position of the awl allows maintaining the position in the operation process. In other words, the entire work flow of the operation will not be disturbed when using the computer assisted surgery system. It should be noted that the illustrations of Figs. 2 to 10 are illustrations in the AP-direction, and that corresponding illustrations may also be obtained in ML-direction to be fed with the computational unit 5.

Fig. 10 illustrates in a similar way the selection of the correct bone screw 210 out of a variety of bone screws, so that the required distances of the bone screw to the surface of the femur head 330 can be maintained. Fig. 10 illustrates two possible lengths of a bone screw 210' and 210c', wherein the system based on the rules for allowable ranges should select the position 210', as the longer bone screw 210c' does not maintain the required distance to the surface of the bone head 330.

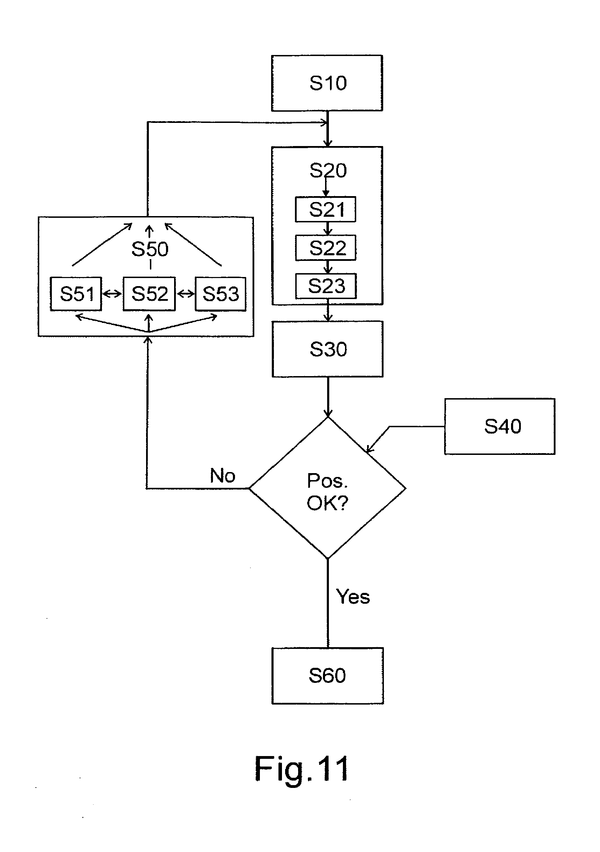

Fig 11 illustrates the method for operating a computer assisted surgery system. The method comprises the positioning S10 of a reference body 100, 200 in relation to an anatomical structure 300, the reference body virtually representing a position of a medical device 200, 210, 220 to be applied to the anatomical structure, detecting S20 a position of the reference body in relation to the anatomical structure, superimposing S30 the anatomical structure with a virtual representation 200'; 210', 220' of a medical device to be applied, based on the detected position of the reference body in relation to the anatomical structure, providing rules S40 for allowable ranges for applying the medical device in relation to the anatomical structure, modifying S50 the position of the reference body and optimizing S60 the virtual position of the medical device to be applied with respect to the anatomical structure so as to obtain a best fit with respect to the rules for allowable ranges. Detecting S20 the positioned reference body 100; 200 in relation to an anatomical structure may further comprise taking two 2-dimensional images from different angles S21, generating a 3-dimensional representation based on the two 2-dimensional image S22, and determining a spatial position of the reference body in relation to the anatomical structure based on the 3-dimensional representation 523. Modifying S50 may comprise rotating S51 and/or displacing S52 of the reference body 100; 200, as well as selecting the medical device 200; 210, 220 out of a predetermined group of a variety of medical devices S53.

In another embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an embodiment of the method as described above.

According to a further embodiment of the present invention, a computer readable medium, such as a CD-ROM, is presented wherein the computer readable medium has a computer program element stored on it which computer program element is described by the preceding section.

However, the computer program may also be presented over a network like the World Wide Web and can be downloaded into the working memory of a data processor from such a network. According to a further embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

It has to be noted that embodiments of the invention are described with reference to different subject matters. In particular, some embodiments are described with reference to apparatus type claims whereas other embodiments are described with reference to method type claims. However, a person skilled in the art will gather from the above and the following description that, unless other notified, in addition to any combination of features belonging to one type of subject matter also any combination between features relating to different subject matters, in particular between features of the apparatus type claims and features of the method type claims is considered to be disclosed with this application.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage.

A computer program may be stored and/or distributed on a suitable medium, such as an optical storage medium or a solid state medium supplied together with or as part of other hardware, but may also be distributed in other forms, such as via the internet or other wired or wireless telecommunication systems.

## Claims

1. Computer assisted surgery system comprising:
a reference body (100; 200) in relation to a femur (300), the reference body virtually representing a position of an intramedullary nail and its bone screw (200; 210, 220) to be applied to the femur as anatomical structure
a detector device (3) being adapted for detecting a position of the reference body in relation to the femur; detecting the positioned reference body in relation to the anatomical structure comprises taking two two-dimensional images from different angles and generating a three-dimensional representation based on the two two-dimensional images, and determining a spatial position of the reference body in relation to the anatomical structure based on the three-dimensional representation,
a data-base (4) including virtual intramedullary nail and bone screw information;
wherein the data-base (4) includes a plurality of data sets for the intramedullary nail and its bone screw (200; 210, 220), wherein the data sets represent a variety of intramedullary nails and its bone screw,
a computation device (5) being adapted for superimposing the representation, with a virtual representation (200'; 210', 220') of an intramedullary nail and its bone screw to be applied based on an output of the detector device, and virtually modifying the position of the reference body and optimizing the virtual position of the intramedullary nail and its bone screw to be applied with respect to the femur so as to obtain a best fit with respect to predetermined rules for allowable ranges for applying the intramedullary nail in relation to the femur, wherein virtually modifying comprises selecting a virtual intramedullary nail (200; 210, 220) from the data base
the nail having an orifice with a specific inclination relative to the longitudinal axis of the nail for receiving the bone screw,
to achieve the correct positioning of the later implanted bone screw wherein selecting is carried out by the computer assisted surgical system when searching for an optimum geometry in the database and proposing the respective type of intramodullary nail.

2. System of claim 1, wherein the system further comprises a medical tool (10) being adapted for positioning the intramedullary nail, wherein the reference body (100: 200) is mountable in a predefined manner to the medical tool.

## Patentansprüche

1. Computergestütztes chirurgisches System, das Folgendes aufweist:
einen Referenzkörper (100; 200) in Beziehung zu einem Oberschenkelknochen (300), wobei der Referenzkörper eine Position eines intramedullären Nagels und seiner Knochenschraube (200, 210, 220), die an dem Oberschenkelknochen als anatomische Struktur angewendet werden sollen, virtuell repräsentiert,
eine Detektorvorrichtung (3), die zum Nachweisen einer Position des Referenzkörpers in Beziehung zu dem Oberschenkelknochen geeignet ist; wobei das Nachweisen des positionierten Referenzkörpers in Beziehung zu der anatomischen Struktur das Aufnehmen von zwei zweidimensionalen Abbildungen aus verschiedenen Winkeln und das Erzeugen einer dreidimensionalen Darstellung auf der Basis der zwei zweidimensionalen Abbildungen und das Bestimmen einer räumlichen Position des Referenzkörpers in Beziehung zu der anatomischen Struktur auf der Basis der dreidimensionalen Darstellung umfasst,
eine Datenbank (4), welche Informationen über virtuelle intramedulläre Nägel und Knochenschrauben enthält;
wobei die Datenbank (4) mehrere Datensätze für den intramedullären Nagel und seine Knochenschraube (200; 210, 220) enthält, wobei die Datensätze eine Vielzahl intramedullärer Nägel und ihrer Knochenschrauben repräsentieren,
eine Verarbeitungseinrichtung (5), die für die Überlagerung der Darstellung mit einer virtuellen Darstellung (200'; 210', 220') eines intramedullären Nagels und seiner Knochenschraube, die angewendet werden sollen, auf der Grundlage einer Ausgabe der Detektorvorrichtung und zum virtuellen Modifizieren der Position des Referenzkörpers und zur Optimierung der virtuellen Position des intramedullären Nagels und seiner Knochenschraube, die angewendet werden sollen, in Beziehung zu dem Oberschenkelknochen geeignet ist, um eine beste Anpassung bezüglich vorgegebener Regeln für zulässige Bereiche zum Anbringen des intramedullären Nagels in Beziehung zu dem Oberschenkelknochen zu erhalten, wobei das virtuelle Modifizieren das Auswählen eines virtuellen intramedullären Nagels (200; 210, 220) aus der Datenbank umfasst,
wobei der Nagel eine Öffnung mit einem spezifischen Neigungswinkel im Verhältnis zu der Längsachse des Nagels zum Aufnehmen der Knochenschraube aufweist,
um die korrekte Positionierung der später implantierten Knochenschraube zu erreichen, wobei das Auswählen durch das computergestützte chirurgische System durchgeführt wird, wenn in der Datenbank nach einer optimalen Geometrie und einem Vorschlag für den entsprechenden Typ des intramedullären Nagels gesucht wird.

2. System nach Anspruch 1, wobei das System ferner ein medizinisches Werkzeug (10) aufweist, das für die Positionierung des intramedullären Nagels geeignet ist, wobei der Referenzkörper (100; 200) in einer vorgegebenen Weise an dem medizinischen Werkzeug befestigbar ist.

## Revendications

1. Système chirurgical assisté par ordinateur comportant :
un corps de référence (100 ; 200) par rapport à un fémur (300), le corps de référence représentant virtuellement une position d'un clou intramédullaire et de sa vis à os (200 ; 210, 220) à appliquer au fémur, sous la forme d'une structure anatomique,
un dispositif de détection (3) adapté pour détecter une position du corps de référence par rapport au fémur, la détection du corps de référence positionné par rapport à la structure anatomique comportant la prise de deux images bidimensionnelles à partir de différents angles et la génération d'une représentation tridimensionnelle sur la base des deux images bidimensionnelles, et déterminer une position spatiale du corps de référence par rapport à la structure anatomique sur la base de la représentation tridimensionnelle,
une base de données (4) incluant des informations de clou intramédullaire et de vis à os virtuels,
dans lequel la base de données (4) inclut une pluralité d'ensemble de données pour le clou intramédullaire et sa vis à os (200 ; 210 ,220), l'ensemble de données représentant une diversité de clous intramédullaires et de leur vis à os,
un dispositif de calcul (5) adapté pour superposer la représentation à une représentation virtuelle (200' ; 210', 220') d'un clou intramédullaire et de sa vis à os à appliquer sur la base d'une sortie du dispositif de détection, et modifier virtuellement la position du corps de référence et optimiser la position virtuelle du clou intramédullaire et de sa vis à os à appliquer par rapport au fémur de manière à obtenir le meilleur ajustement par rapport à des règles prédéterminées concernant des plages admissibles pour appliquer le clou intramédullaire par rapport au fémur, dans lequel la modification virtuelle comporte la sélection d'un clou intramédullaire virtuel (200 ; 210, 220) dans la base de données, le clou ayant un orifice avec une inclinaison spécifique par rapport à l'axe longitudinal du clou pour recevoir la vis à os, afin d'obtenir le positionnement correct de la dernière vis à os implantée, la sélection étant effectuée par le système chirurgical assisté par ordinateur lors de la recherche d'une géométrie optimale dans la base de données et la proposition du type respectif de clou intramédullaire.

2. Système selon la revendication 1, dans lequel le système comporte en outre un outil médical (10) adapté pour positionner le clou intramédullaire, dans lequel le corps de référence (100 ; 200) peut être monté de manière prédéfinie sur l'outil médical.
